**Europäisches Patentamt**

**European Patent Office**

**Office européen des brevets**

(19)

(11) Veröffentlichungsnummer : **0 407 495 B1**

(12) # EUROPÄISCHE PATENTSCHRIFT

(45) Veröffentlichungstag der Patentschrift :
**12.05.93 Patentblatt 93/19**

(51) Int. Cl.$^5$ : **B01J 19/12,** // **A61L2/10, C02F1/32**

(21) Anmeldenummer : **89911764.2**

(22) Anmeldetag : **25.10.89**

(86) Internationale Anmeldenummer :
**PCT/DE89/00691**

(87) Internationale Veröffentlichungsnummer :
**WO 90/04454 03.05.90 Gazette 90/10**

(54) ## ANORDNUNG ZUR BESTRAHLUNG VON LAUFENDEN FLÜSSIGKEITEN UND/ODER GASEN MIT ULTRAVIOLETT.

(30) Priorität : **26.10.88 DE 3836494**

(43) Veröffentlichungstag der Anmeldung :
**16.01.91 Patentblatt 91/03**

(45) Bekanntmachung des Hinweises auf die
Patenterteilung :
**12.05.93 Patentblatt 93/19**

(84) Benannte Vertragsstaaten :
**AT BE CH DE FR GB IT LI LU NL SE**

(56) Entgegenhaltungen :
**FR-A- 2 428 323**
**US-A- 3 657 087**
**US-A- 4 367 410**
**Patent Abstracts of Japan, vol. 13, Nr. 137**
**(C-852), 5 April 1989 & JP-A-63302940**

(73) Patentinhaber : **WEDECO GESELLSCHAFT FÜR ENTKEIMUNGSANLAGEN MBH
Daimlerstrasse 5
W-4900 Herford (DE)**

(72) Erfinder : **WEDEKAMP, Horst
Elverdisserstrasse 92
W-4900 Herford (DE)**

(74) Vertreter : **Patentanwälte Thömen & Körner
Zeppelinstrasse 5
W-3000 Hannover 1 (DE)**

## Beschreibung

Die Erfindung betrifft eine Vorrichtung gemäß dem Oberbegriff des Patentanspruchs 1 sowie gemäß dem Oberbegriff des Patentanspruchs 12.

Solche zur UV-Lichtbestrahlung von Medien bestimmte Vorrichtungen sind allgemein bekannt. Dabei besteht der Wunsch, daß die Vorrichtungen zur UV-Lichtbestrahlung von Medien im Vergleich zu möglichen chemischen Verfahren bei gleicher Wirtschaftlichkeit möglichst wirkungsvoller im Behandlungseffekt sein sollen. Dies setzt eine hohe UV-Licht-Raum-Zeit-Ausbeute bei geringstem Energie- und Apperateaufwand voraus.

Für die Realisierung dieser Vorrichtungen wird in der Regel eine Kombination zwischen dem Bestrahlungsraum und der UV-Lichtquellenanordnung angestrebt, bei der jedes Volumenelement des Mediums mit einer möglichst gleich großen UV-Dosis bestrahlt wird. Der Idealfall zur Erzielung eines höchsten Wirkungsgrades besteht darin, daß die Durchschnittsdosis gleich der Mindestdosis ist. Zur Realisierung dieser Zielsetzung gilt es, folgende Kriterien zu optimieren:

1. Vermeidung von Strahlungsverlusten;
2. Erzielung einer möglichst gleichmäßigen Bestrahlungsstärke;
3. Erzielung einer möglichst gleichmäßigen Kontaktzeit bzw. Strömungsgeschwindigkeit.

Die Vorraussetzungen gemäß Ziffer 2 und 3 sind oft nur durch aufwendige Hilfseinrichtungen zur Verwirbelung des zu entkeimenden bzw. zu behandelnden Mediums zu erfüllen.

Aus der US-A-3 657 087 ist eine Vorrichtung zum Bestrahlen von strömenden Medien mit UV-Licht bekannt, die aus einem Gehäuse mit Zu- und Abführungsöffnungen und einer oder mehreren UV-Lichtquellen besteht. Die UV-Lichtquellen befinden sich unmittelbar an den Stirnwänden eines länglichen Gehäuses. Der Zufluß und Abfluß von Medium kann daher nicht ebenfalls über die Stirnwände des Gehäuses vorgenommen werden, sondern wird vielmehr durch seitliche Stutzen bewirkt.

Da das strömende, zu entkeimende Medium bestrebt ist, auf dem kürzesten Weg vom Einlaß zum Auslaß zu gelangen, wird ein Teil des Medium-Stromes bereits unmittelbar nach dem Übertritt von dem Stutzen in das Gehäuse in die Achsrichtung des Gehäuses einschwenken und daher nicht in unmittelbare Nähe der UV-Lichtquellen gelangen. Dieser Anteil des Mediums erhält daher eine geringere UV-Strahlendosis als das Medium, das in den den Stutzen gegenüberliegenden Ecken des Gehäuses längere Zeit verweilt.

Der Erfindung liegt die Aufgabe zugrunde, eine Vorrichtung zum Bestrahlen von strömenden Flüssigkeiten und/oder Gasen mit UV-Licht dahingehend zu verbessern, daß bei einfachem Aufbau Strahlungsverluste verringert werden, um eine möglichst hohe gleichmäßige Bestrahlungsstärke zu erreichen, und daß weiterhin gleichmäßige Kontaktzeiten erzielbar sind.

Gemäß einer ersten Alternative erfolgt die Lösung dieser Aufgabe bei der im Oberbegriff des Anspruchs 1 genannten Vorrichtung durch die Merkmale des kennzeichnenden Teils des Anspruchs 1.

Die neue Vorrichtung ist für den Einbau in Rohrleitungen konzipiert und verfügt hierfür über eine neuartige Strahlungsverteilung, durch welche die voranstehend genannten Kriterien weitgehend erfüllt werden können. Die Verwendung von Rohrleitungen bedeutet, daß es sich um ein Drucksystem handelt, bei welchem das Medium (Flüssigkeit und/oder Gase) mittels Druck durch die Rohrleitungen geleitet wird.

In der Mitte des Gehäuses und quer zur Strömungsrichtung des Mediums sind wahlweise ein einzelnes oder mehrere Quarzschutzrohre, die jeweils mit einer stabförmigen UV-Lichtquelle bestückt sind, angeordnet. Die Bestückung mit einer oder mehreren UV-Lichtquellen erfolgt in Abhängigkeit der gewünschten Durchsatzmenge. Für große Durchsatzleistungen wird eine Mehrzahl von UV-Lichtquellen nebeneinander und in maximal zwei Reihen eingesetzt. Bei der Anordnung in zwei Reihen sind die UV-Lichtquellen in Fließrichtung des Mediums gegeneinander versetzt.

Die UV-Lichtquellen haben vorzugsweise einen flachovalen Querschnitt und im Vergleich zu bekannten UV-Lichtquellen mit rundem Querschnitt neben höheren elektrischen Leistungen eine besondere Strahlungscharakteristik. Bei einem Flachstrahler - also bei einer UV-Lichtquelle mit einem flach-ovalen Querschnitt - wird der Strahlungfluß zu 3/4 über die Breitseite und nur zu 1/4 über die Schmalseite emittiert. Die Ausstrahlung über die Breitseite erfolgt außerdem wesentlich stärker gebündelt, als bei UV-Lichtquellen mit rundem Querschnitt.

Hieraus ergibt sich eine im Maximum nach zwei Seiten gerichtete UV-Emission. Durch die Anordnung einer einzelnen oder mehrerer UV-Lichtquellen (Flachstrahler) in einer Ebene quer zur Strömungsrichtung und benachbart zu den Anschlußöffnungen des Gehäuses bzw. der angeschlossenen Rohrleitungen gelangt das Maximum der UV-Emission im weitgehend parallelen Strahlengang in die angeschlossenen Rohrleitungen, und zwar zu gleichen Teilen sowohl in das zu- als auch in das abströmende Medium.

Da die ankommende und abgehende Rohrleitung gradlinig mit der Strahlungsrichtung verlaufen soll, kann die Strahlung verlustlos, d.h., bis zu ihrer völligen Absorption im Medium eindringen. Dies ist bei den bekannten Vorrichtungen bislang nicht gegeben.

EP 0 407 495 B1

Durch die AT-PS 362 076 ist zwar eine Vorrichtung zum Entkeimen von Flüssigkeiten bekannt, bei welcher ein Strahlungsverlust mit Hilfe von Reflektoren zwar weitgehend vermieden wird, trotzdem geht aber ein Großteil der Strahlung nach Durchstrahlen der Bestrahlungskammer verloren.

Solche Nachteile der bekannten Vorrichtungen entfallen bei der erfindungsgemäßen Vorrichtung (die auch als UV-Lichtschranke bezeichnet wird). Aufgrund ihrer Bauform und der gerichteten Strahlungsverteilung werden Wandverluste weitgehend ausgeglichen. Bei einer hohen Strahlendichte ist eine nahezu homogen Strahlungsverteilung im zu behandelnden Medium sowie ein verlustarmer Transfer des Strahlungsflusses, d.h., eine fast völlig Absorbtion der UV-Emission im Medium gewährleistet.

Durch die gerichtete und weitgehend parallele Ausrichtung der UV-Emission wird der mathematische Grenzfall einer ausgedehnten ebenen Strahlenguelle angenähert erreicht, und die geometrisch bedingte Abschwächung der Bestrahlungsstärke bei zunehmendem Abstand von der Strahlenguelle wird weitgehend vermieden.

Aus der Parallelität von Fließrichtung und Strahlungsrichtung folgt, daß sich die einem Volumenelement des Mediums applizierte Dosis aus dem Quotienten von Bestrahlungsstärke und Fließgeschwindigkeit des Mediums, integriert über die gesamte Länge der Rohrleitung, ergibt.

Die Abhängigkeit der applizierten Dosis vom bestand zur Achse ist durch Multiplikation mit dem Verhältnis der radialen Verteilung von Bestrahlungsstärke und Geschwindigkeit zu berücksichtigen. Räumliche Unterschiede der Bestrahlungsstärke in axialer Richtung werden also durch Mittelwertbildung über den Weg des Volumenelements ausgeglichen. Durch diese Mittelwertbildung wird die Abhängigkeit der Bestrahlungsstärke vom axialen Abstand zur Strahlenquelle eliminiert. Ein hohes Maß an Gleichverteilung der jedem Volumenelement applizierten Dosis ist die Folge.

Die radiale Verteilung der Bestrahlungsstärke besitzt in der Rohrmitte ein flaches Maximum und schwächt sich bei der Annäherung an den Rohrrand ab. Die radiale Verteilung der Geschwindigkeit besitzt ein ähnliches Profil. Modellrechnungen ergeben, daß der Quotient beider Verteilungsfunktionen je nach Fließgeschwindigkeit und Strahleranordnung über den gesamten Rohrquerschnitt innerhalb von +/- 10% konstant ist.

In einer Vorrichtung nach der Erfindung wird im Gegensatz zu herkömmlichen UV-Bestrahlungsanlagen erstmalig jedem Volumenelement innerhalb weniger Prozentpunkte Schwankungsbreite die gleiche UV-Dosis appliziert.

Computersimulationen haben unter Berücksichtigung von Randeffekten, Querschnittsveränderungen, UV-Transmission des Wassers sowie der Anordnung der UV-Strahlungsquellen die in der nachstehenden Tabelle aufgeführte, beispielhafte Leistungsfähigkeit einer Vorrichtung nach der Erfindung ergeben. Die Tabelle zeigt einen rechnerischen Vergleich der Leitungsfähigkeit einer bekannten Vorrichtung vom Typ "4 - B" der Anmelderin mit einer Vorrichtung nach der Erfindung (bei einer Dosis von 35 mWs/cm$^2$).

| Vorrichtung | Typ "4 - B" | Erfindung |
|---|---|---|
| Anzahl der UV-Strahler | 4 | 5 |
| effektive UV-Leistung (254 nm) | 184 W | 158 W |
| Durchsatz: | | |
| T (1 cm) = 96% | 80 m$^3$/h | 106 m$^3$/h |
| T (1 cm) = 90% | 59 m$^3$/h | 74 m$^3$/h |

3

Vorrichtung nach der Erfindung: Durchmesser der Rohrleitungen 250 mm; Netto-Querschnitt 511 cm². Reaktorvolumen bei der bekannten Vorrichtung: 31 Liter.

Eine Vorrichtung nach der Erfindung zeichnet sich durch einen um etwa 50% höheren Wirkungsgrad gegenüber einer herkömmlichen Vorrichtung mit Strahleranordnung in Fließrichtung aus.

Die gerichtete Strahlung der UV-Emission, die Anordnung der UV-Lichtquellen zueinander und quer zur Strömungsrichtung sowie die angepaßte Gehäuseform der Vorrichtung ergeben also einen bislang nicht erreichten Wirkungsgrad im Transfer der UV-Lichtstrahlung an das zu behandelnde Medium, der mit bekannten Vorrichtungen nicht erreicht werden kann.

Ein besonderer Vorteil der Erfindung besteht darin, daß die neue Vorrichtung geeignet ist, nachträglich in bereits bestehende Rohrleitungen eingebaut zu werden, wobei sich die Vorrichtung einfach als Zwischenstück einfügen läßt.

Gemäß einer anderen Alternative wird die der Erfindung zugrunde liegende Aufgabe bei einer Vorrichtung nach dem Oberbegriff des Patentanspruchs 12 durch die im kennzeichnenden Teil des Anspruchs 12 angegebenen Merkmale gelöst.

Hierbei ist keine Rohrleitung vorhanden, vielmehr wird ein von einer Flüssigkeit durchflossenes Gerinne verwendet, wie es bei Kläranlagen eingesetzt wird.

Es handelt sich hier also um ein druckloses System, wobei das Gerinne als nach oben offenes Gehäuse aufzufassen ist.

Diese Lösungsform führt ebenfalls zu den voranstehend geschilderten Vorteilen bei der Entkeimung von Flüssigkeiten mittels UV-Strahlung, die gerichtet ist und sich gradlinig in Richtung des ebenfalls gradlinigen Gerinnes erstreckt.

Vorzugsweise lassen sich mehrere in einer und auch mehreren Reihen zusammengefaßte UV-Lichtquellen nach Art eines Strahlermoduls ausgestalten, welches einfach handhabbar und somit auch einfach von oben her in das Gerinne einsetzbar ist. Bei Bedarf können in bestimmten Abständen voneinander mehrere solcher Strahlermodule in das Gerinne eingesetzt werden.

Nachfolgend wird die Erfindung anhand der in der Zeichnung dargestellten Ausführungsbeispiele näher erläutert. Es zeigen:

Fig. 1       eine schematische Darstellung einer in eine Rohrleitung eingefügten Vorrichtung,

Fig. 2       eine Draufsicht der Vorrichtung gemäß Fig. 1,

Fig. 3       eine schematische Darstellung einer anderen Vorrichtung mit zwei Reihen von UV-Lichtquellen mit Reflektoren,

Fig. 4       eine weitere Ausführungsform, bei welcher sich der Gehäusequerschnitt der Vorrichtung zu den Anschlußstellen der Rohrleitung verjüngt,

Fig. 5       eine perspektivische Darstellung eines Gerinnes, und

Fig. 6       eine Draufsicht des Gerinnes gemäß Fig. 5, mit darin eingesetzten Strahlermodulen.

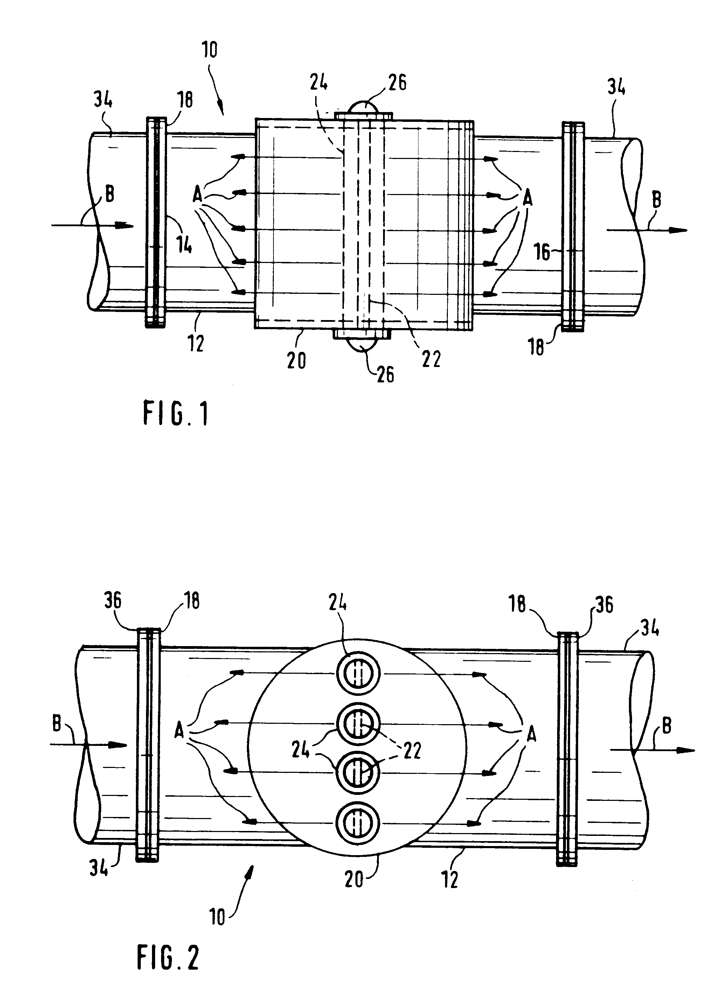

Gemäß Fig. 1 und 2 ist eine Vorrichtung 10 mit UV-Lichtquellen 22 in eine Rohrleitung 34 eingefügt, die von einer Flüssigkeit in Richtung der Pfeile B durchströmt wird. Die Verbindung der Vorrichtung 10 erfolgt über die beiden Gehäuseanschlüsse 18 und die dazugehörigen Rohranschlüsse 36.

Die Vorrichtung 10 mit ihrem Gehäuse 12 besitzt eine Haltevorrichtung 20, welche vier in einer Reihe quer zur Strömungsrichtung B angeordnete UV-Lichtquellen trägt, die als Flachstrahler mit flachovalem Querschnitt ausgebildet und jeweils von einem Schutzrohr 24 umgeben sind. Außerhalb der Haltevorrichtung 20 verlaufen die elektrischen Anschlüsse 26 der Flachstrahler 22.

Eine in Strömungsrichtung B in der Rohrleitung 34 fließende Flüssigkeit gelangt durch eine Zuführungsöffnung 14 in das Gehäuse 12 der Vorrichtung, und durch eine Abführungsöffnung 16 fließt die Flüssigkeit über den rechten Teil der Rohrleitung 34 ab. Die Flüssigkeit durchströmt also das Gehäuse 12 mit den Flachstrahlern 22, die in einer Reihe quer zur Strömungsrichtung B angeordnet sind.

Aufgrund seiner an sich bekannten Eigenschaft erzeugt jeder Flachstrahler 22 eine Strahlung mit einer bevorzugten Strahlungsrichtung A. Das Maximum der UV-Licht-Ausstrahlung ist also gerichtet und erstreckt sich in Strömungsrichtung bzw. in entgegengesetzter Strömungsrichtung B der Flüssigkeit. Somit kann sich die UV-Strahlung über das Gehäuse 12 hinaus in die geradlinig verlaufende Rohrleitung 34 zu beiden Seiten der Vorrichtung 10 erstrecken. Das Gehäuse 12 und die Rohrleitung 34 besitzen hier jeweils den gleichen Querschnitt.

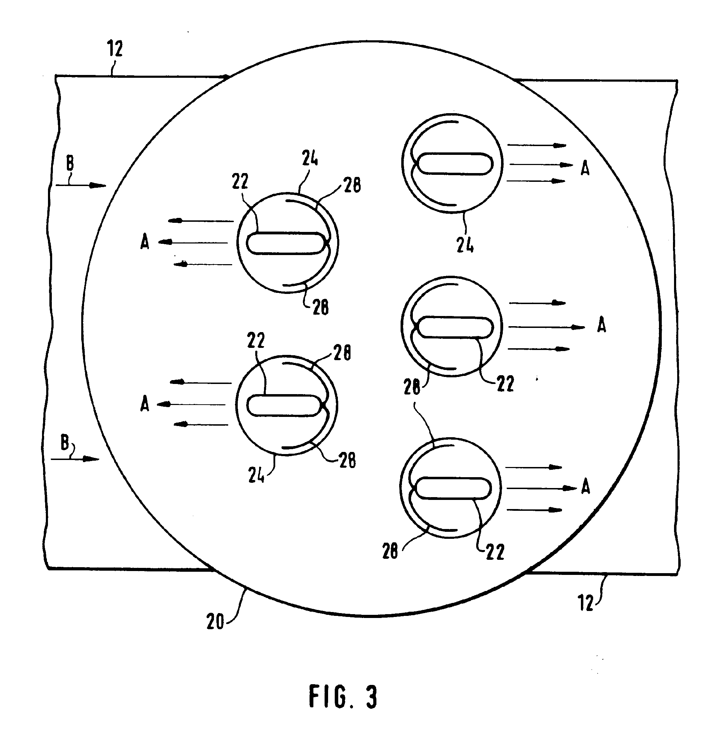

Bei der Ausführungsform einer erfindungsgemäßen Vorrichtung gemäß Fig. 3 sind zwei quer zur Strömungsrichtung B angeordnete Reihen von UV-Lichtquellen 22 vorgesehen, die zur Unterstützung und Verbesserung einer gerichteten Ausstrahlung A mit Reflektoren 28 versehen sind. Durch diese Reflektoren 28 wird die Strahlung der einzelnen Flachstrahler 22 so umgelenkt und beeinflußt, daß sie - wie in Fig. 3 durch die Pfeile A angedeutet - verstärkt jeweils nur entweder nach links oder nach rechts eine gerichtete Strahlung abgeben. Grundsätzlich sind auch andere Reflektortypen denkbar, und es lassen sich auch UV-Lichtquellen mit

4

rundem Querschnitt verwenden, bei denen durch geeignete Reflektoren eine gerichtete Strahlung erzeugt werden kann. Zu beachten ist bei den in Fig. 3 dargestellten Flachstrahlern 22, daß sich ihre Breitseiten hier nicht quer, sondern parallel zur Strömungsrichtung B erstrecken, weil dadurch in Verbindung mit den Reflektoren 28 eine optimale gerichtete UV-Lichtstrahlung A erzielt werden kann.

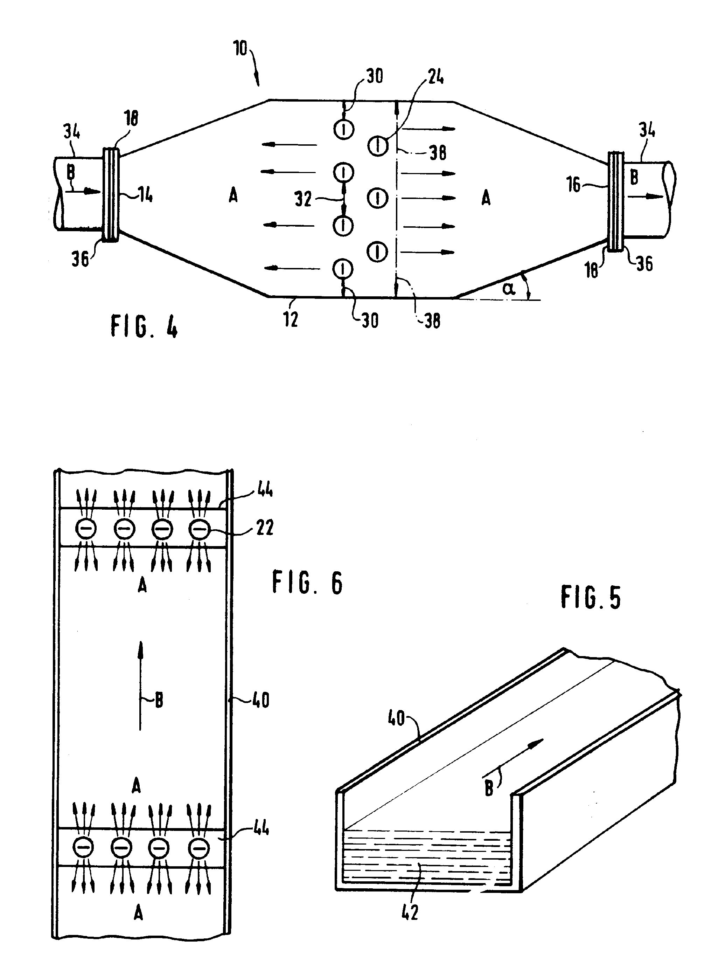

In Fig. 4 ist eine Ausführungsform dargestellt, bei welcher der mittige Gehäusequerschnitt 38 im Bereich der Flachstrahler 22 größer ausgebildet ist, als der Querschnitt der angeschlossenen Rohrleitung 34. Durch diese Maßnahme läßt sich die Verweildauer der Flüssigkeit innerhalb des Gehäuses 12 erhöhen, was zu einer noch besseren Entkeimung führt. Die konische Verjüngung des Gehäusequerschnittes 38 zu den beiden Gehäuseanschlüssen 18 erfolgt vorzugsweise unter einem Winkel α, der nicht größer als 45° ist. Ferner wird das Verhältnis des Gehäusequerschnittes 38 zum Querschnitt der angeschlossenen Rohrleitung 34 nicht größer als 1:1,5 gewählt. Mit diesen Randbedingungen lassen sich besonders hervorragende Ergebnisse erzielen.

Von Vorteil sind auch noch folgende Maßnahmen. Der in Fig. 4 zu erkennende Abstand 30 zwischen dem Schutzrohr der äußeren UV-Lichtquellen 22 einer Reihe und der Gehäusewand des Gehäuses 12 ist so klein bemessen, daß die Bestrahlungsstärke der auf die Gehäusewand gerichteten Strahlung unmittelbar vor dem Auftreffen auf die Gehäusewand immer noch etwa 50 % der von der betreffenden UV-Lichtquelle erzeugten Bestrahlungsstärke beträgt. Ferner sind die Abstände 32 zwischen den Schutzrohren der benachbarten UV-Lichtquellen 22 mindestens doppelt so groß gewählt, wie die voranstehend erwähnten Abstände 30.

Die Flachstrahler 22 strahlen nämlich mit ihrem Maximum nicht nur in Richtung A, sondern - wenn auch wesentlich geringer - quer zur Strömungsrichtung B. Durch die vorgegebene Bemessung der Abstände 30 und 32 ist dabei gewährleistet, daß auch zwischen den benachbarten UV-Lichtquellen noch eine hinreichende Bestrahlungsstärke gegeben ist, daß also das hier vorbeifließende Medium noch in gewünschtem Maße der UV-Strahlung ausgesetzt ist.

Die soweit beschriebenen Vorrichtungen gemäß Fig. 1 - 4 betreffen einen Aufbau mit einer Rohrleitung. Dabei handelt es sich um ein sogenanntes Drucksystem, bei welchem die Flüssigkeit unter Druck durch die Rohrleitung 34 und das Gehäuse 12 der Vorrichtung 10 geführt ist.

Die Erfindung läßt sich aber mit Vorteil auch bei drucklosen Systemen einsetzen. Hierzu zeigt Fig. 5 ein bei Klärwerken verwendetes nach oben hin offenes Gerinne 40, welches nach Art eines Grabens ausgebildet ist und von einer Flüssigkeit 42 durchflossen wird.

Bei einem solchen drucklosen System mit einem Gerinne 40 läßt sich nach der Erfindung gemäß der Darstellung in Fig. 6 von oben her in einfacher Weise ein Strahlermodul 44 einsetzen, welches mehrere in einer Reihe quer zur Strömungsrichtung angeordnete UV-Flachstrahler umfaßt. Dabei tauchen die UV-Flachstrahler 22 mit ihren üblichen Schutzrohren in die Flüssigkeit 42 ein. Die UV-Licht-Ausstrahlung A ist wiederum gerichtet und verläuft mit ihren Maxima in die Richtungen B der zu- und abströmenden Flüssigkeit 42.

Bei Bedarf können in Abständen voneinander auch mehrere Strahlermodule 44 eingesetzt werden.

## Patentansprüche

1. Vorrichtung zum Bestrahlen von strömenden Flüssigkeiten und/oder Gasen mit UV-Licht, bestehend aus einem Gehäuse mit Zu- und Abführungsöffnungen und einer oder mehrerer in UV-durchlässigen Schutzrohren angeordneten UV-Lichtquellen, dadurch gekennzeichnet, daß die UV-Lichtquellen (22) quer zur Strömungsrichtung im axial durchströmten Bereich des Gehäuses (12) angeordnet sind und daß die UV-Licht-Ausstrahlung (A) gerichtet ist und mit ihren Maxima in die Richtungen (B) das axial zu- und abströmenden Mediums erfolgt.

2. Vorrichtung nach Anspruch 1, dadurch gekennzeichnet, daß die UV-Lichtquellen (22) zur Erzeugung einer gerichteten Strahlungsverteilung (A) und erhöhten Strahlendichte einen flach-ovalen Querschnitt aufweisen und/oder Schutzrohre (24) mit lichtumlenkenden Eigenschaften, wie z.B. durch Riffelungen und/oder Lichtreflektionen (28) verwendet werden.

3. Vorrichtung nach einem der vorhergehenden Ansprüche 1 - 2, dadurch gekennzeichnet, daß eine Mehrzahl von UV-Lichtquellen (22) nebeneinander in einer oder mehreren Reihen quer zur Strömungsrichtung (B) eingesetzt sind.

4. Vorrichtung nach einem der vorhergehenden Ansprüche 1 - 3, dadurch gekennzeichnet, daß die Abstände (30) zwischen den seitlichen Gehäusewänden der Vorrichtung (10) und den jeweils nächsten UV-Lichtquellen so klein bemessen sind, daß die Bestrahlungsstärke (Strahlendichte) längs dieses Abstandes nicht mehr als 50% abnimmt.

**5.** Vorrichtung nach Anspruch 4, dadurch gekennzeichnet, daß bei dem Einsatz mehrerer UV-Lichtquellen (22) die Abstände (32) der UV-Lichtquellen (22) einer Reihe zueinander mindestens doppelt so groß sind, wie die Abstände (30) zu den seitlichen Gehäusewänden des Gehäuses (12) der Vorrichtung (10).

**6.** Vorrichtung nach einem der vorhergehenden Ansprüche 1 - 5, dadurch gekennzeichnet, daß der Querschnitt der Zuführungsöffnung (14) und der Abführungsöffnung (16) gleich dem Querschnitt des Gehäuses (12) ist.

**7.** Vorrichtung nach einem der vorhergehenden Ansprüche 1 - 5, dadurch gekennzeichnet, daß der Querschnitt der Zuführungsöffnung (14) und der Abführungsöffnung (16) halb so groß ist, wie die gesamte Querschnittsfläche, die sich aus den freien Durchgängen bzw. Abständen (32) zwischen den einzelenen UV-Lichtquellen (22) mit ihren Schutzrohren (24) sowie aus den Abständen (30) zu den seitlichen Gehäusewänden ergibt.

**8.** Vorrichtung nach einem der vorhergehenden Ansprüche 1 - 5, dadurch gekennzeichnet, daß der Querschnitt (38) des Gehäuses (12) größer als der Querschnitt der Zuführungsöffnung (14) und der Abführungsöffnung (16) ist, und daß der Gehäusequerschnitt (38) an seinen beiden Enden sich auf den Querschnitt der Zuführungsöffnung (14) und der Abführungsöffnung (16) verjüngt.

**9.** Vorrichtung nach Anspruch 8, dadurch gekennzeichnet, daß sich der Gehäusequerschnitt (38) vorzugsweise konisch verjüngt, wobei das Verhältnis des mittigen unverjüngten Gehäusequerschnittes (38) zum Querschnitt der mit unverändertem Radius angeschlossenen Rohrleitungen (34) nicht größer als 1:1,5 ist, und daß der Verjüngungswinkel ($\alpha$) nicht größer als 45° ist.

**10.** Vorrichtung nach einem der vorhergehenden Ansprüche 1 - 9, dadurch gekennzeichnet, daß die Rohrleitungen (34) für das zu behandelnde Medium sich in den Richtungen der maximalen Lichtausstrahlung der UV-LIchtquellen (22) erstrecken.

**11.** Vorrichtung nach einem der vorhergehenden Ansprüche 1 - 10, dadurch gekennzeichnet, daß temperaturstabilisierte UV-Lichtquellen (22) eingesetzt sind.

**12.** Vorrichtung zum Bestrahlen von strömenden Flüssigkeiten mit UV-Licht, bestehend aus einem Gehäuse mit Zu- und Ablauföffnungen und einer oder mehrerer in UV-durchlässigen Schutzrohren angeordneten UV-Lichtquellen, dadurch gekennzeichnet, daß das Gehäuse als nach oben offenes Gehäuse in Form eines von Flüssigkeit (42) durchflossenen Gerinnes (40) ausgebildet ist, daß die UV-Lichtquellen (22) quer zur Strömungsrichtung innerhalb des axial durchströmten Bereichs des Gerinnes (40) angeordnet sind und daß die UV-Licht-Ausstrahlung (A) gerichtet ist und mit ihrem Maximum in die Richtungen (B) des axial zu- und abströmenden Mediums erfolgt.

**13.** Vorrichtung nach Anspruch 12, dadurch gekennzeichnet, daß eine Mehrzahl von UV-Lichtquellen (22) nebeneinander in einer oder mehreren Reihen quer zur Strömungsrichtung (B) eingesetzt sind, wobei die UV-Lichtquellen zu einem Strahlermodul (44) als Baueinheit zusammengefaßt sind.

**14.** Vorrichtung nach Anspruch 13, dadurch gekennzeichnet, daß in Abständen längs des Gerinnes (40) Strahlermodule (44) eingesetzt sind.

## Claims

**1.** Device for irradiating flowing liquids and/or gases with ultraviolet light, consisting of a housing with inlet and outlet openings and one or more UV light sources disposed in protective tubes transparent to UV light, characterized in that the UV light sources (22) are disposed transversely to the flow direction in the region of the housing (12) through which the flow is axial, and that the UV light emission (A) is orientated and lies with its maxima in the directions (B) of the axially inflowing and outflowing medium.

**2.** Device according to Claim 1, characterized in that the UV light sources (22), for the purpose of producing a directed radiation distribution (A) and increased radiation density, have a flat-oval cross-section and/or protective tubes (24) having light deflecting properties, such as by corrugations and/or light reflections (28), are used.

3. Device according to one of the preceding Claims 1 to 2, characterized in that a plurality of UV light sources (22) adjacent to one another in one or more rows transversely to the flow direction (B) are used.

4. Device according to one of the preceding Claims 1 to 3, characterized in that the spacings (30) between the lateral housing walls of the device (10) and the nearest UV light sources in each case are of sufficiently small dimensions for the radiation density not to decrease by more than 50 % along this spacing.

5. Device according to Claim 4, characterized in that, where a plurality of UV light sources (22) is used, the distances (32) from one another of the UV light sources (22) of a row are at least twice as large as the distances (30) from the lateral housing walls of the housing (12) of the device (10).

6. Device according to one of the preceding Claims 1 to 5, characterized in that the cross-section of the inlet opening (14) and of the outlet opening (16) is equal to the cross-section of the housing (12).

7. Device according to one of the preceding Claims 1 to 5, characterized in that the cross-section of the inlet opening (14) and of the outlet opening (16) is half as large as the total cross-sectional area which is obtained from the free passages or spacings (32) between the individual UV light sources (22) with their protective tubes (24) and also from the spacings (30) from the lateral housing walls.

8. Device according to one of the preceding Claims 1 to 5, characterized in that the cross-section (38) of the housing (12) is larger than the cross-section of the inlet opening (14) and of the outlet opening (16), and that the housing cross-section (38) tapers at its two ends to the cross-section of the inlet opening (14) and of the outlet opening (16).

9. Device according to Claim 8, characterized in that the housing cross-section (38) tapers preferably conically, the ratio of the middle, untapered housing cross-section (38) to the cross-section of the pipes (34) connected with unchanged radius being not greater than 1:1.5, and that the angle of taper ($\alpha$) is not greater than 45°.

10. Device according to one of the preceding Claims 1 to 9, characterized in that the pipes (34) for the medium to be treated extend in the directions of the maximum light emission from the UV light sources (22).

11. Device according to one of the preceding Claims 1 to 10, characterized in that temperature-stabilized UV light sources (22) are used.

12. Device for irradiating flowing liquids with ultraviolet light, consisting of a housing having inlet and outlet openings and of one or more UV light sources disposed in protective tubes transparent to UV light, characterized in that the housing is constructed as an upwardly open housing in the form of a channel (40) through which liquid (42) flows, that the UV light sources (22) are disposed transversely to the flow direction within the region of the channel (40) through which the flow is axial, and in that the UV light emission (A) is orientated and lies with its maximum in the directions (B) of the axially inflowing and outflowing medium .

13. Device according to Claim 12, characterized in that a plurality of UV light sources (22) adjacent to one another in one or more rows transversely to the flow direction (B) are used, the UV light sources being combined to a radiator module (44) in the form of one unit.

14. Device according to Claim 13, characterized in that radiator modules (44) are used at spacings along the channel (40).

**Revendications**

1. Dispositif d'exposition de liquides et/ou gaz circulant aux rayons ultraviolets, constitué d'un boîtier, avec des ouvertures d'entrée et de sortie, et d'une ou de plusieurs sources de lumière ultraviolette dans des tubes protecteurs perméables aux UV, caractérisé en ce que les sources de lumière ultraviolette (22) sont perpendiculaires à la direction d'écoulement dans la zone du boîtier (12) que le milieu traverse axialement et en ce que le rayonnement de lumière UV (A) est dirigé et s'effectue, avec ses maxima, dans les directions (B) du milieu passant axialement de l'entrée à la sortie.

**2.** Dispositif suivant la revendication 1, caractérisé en ce que les sources de lumière UV (22) présentent une section plate- ovale pour obtenir une répartition du rayonnement dirigé (A), et une densité plus grande des rayons et/ou sont utilisés des tubes protecteurs (24) ayant des propriétés diffusant la lumière, par exemple, au moyen de cannelages et/ou de réflexions de lumière (28).

**3.** Dispositif suivant l'une des revendications précédentes 1 et 2, caractérisé en ce que plusieurs sources de lumière UV (22) sont utilisées, les unes à côté des autres, en une ou plusieurs rangées perpendiculaires à la direction d'écoulement (B).

**4.** Dispositif suivant l'une des revendications précédentes 1 et 3, caractérisé en ce que les distances (30) entre les parois latérales du boîtier du dispositif (10) et les sources de lumière UV respectives les plus proches sont de dimension assez petites pour que l'intensité du rayonnement (densité des rayons) le long de cet écartement ne soit pas réduit de plus de 50 %.

**5.** Dispositif suivant la revendication 4, caractérisé en ce que, en utilisant plusieurs sources de lumière UV (22), les distances (32) entre les sources de lumière UV (22) d'une même rangée représentent au moins le double des distances (30) des parois latérales du boîtier (12) du dispositif (10).

**6.** Dispositif suivant l'une des revendications 1 à 5, caractérisé en ce que la section transversale des ouvertures d'entrée (14) et de sortie (16) est identique à la section transversale du boîtier (12).

**7.** Dispositif suivant l'une des revendications précédentes 1 à 5, caractérisé en ce que la section transversale des ouvertures d'entrée (14) et de sortie (16) est moitié moins grande que la superficie de section totale qui résulte des passages libres ou des distances (32) entre les différentes sources de lumière UV (22) et leurs tubes protecteurs (24), et des distances (30) des parois latérales du boîtier.

**8.** Dispositif suivant l'une des revendications précédentes 1 à 5, caractérisé en ce que la section transversale (38) du boîtier (12) est plus grande que la section transversale des ouvertures d'entrée (16) et de sortie (18) et que la section transversale du boîtier (38) diminue aux extrémités jusqu'à ce qu'elle corresponde à la section transversale des ouvertures d'entrée (14) et de sortie (16).

**9.** Dispositif suivant la revendication 8, caractérisé en ce que la section transversale du boîtier (38) diminue de préférence de façon conique, le rapport entre la section transversale du boîtier (38) non diminuée en non milieu et la section transversale des conduits tubulaires (34) raccordés avec un rayon non modifié ne dépasse pas 1:1,5 et en ce que l'angle de diminution ($\gamma$) ne dépasse pas 45°.

**10.** Dispositif suivant l'une des revendications 1 à 9, caractérisé en ce que les conduits tubulaires (34) pour le milieu à traiter sont dans les directions du rayonnement maximum des sources de lumière UV (22).

**11.** Dispositif suivant l'une des revendications précédentes 1 à 10, caractérisé en ce que les sources de lumière UV (22) sont stabilisées en température.

**12.** Dispositif d'exposition de liquides circulants à la lumière UV, constitué d'un boîtier avec des ouvertures d'entrée et de sortie, et d'une ou de plusieurs sources de lumière UV disposées dans des tubes protecteurs perméables aux UV, caractérisé en ce que le boîtier est conçu comme un boîtier ouvert on haut sous forme d'un canal (40) traversé par un liquide (42), en ce que les sources de lumière UV (22) sont perpendiculaires à la direction d'écoulement à l'intérieur de la zone du canal (40), que le milieu traverse axialement et en ce que le rayonnement de lumière UV (A) est dirigé et s'effectue, avec ses maxima, dans les directions (B) du milieu passant axialement de l'entrée à la sortie.

**13.** Dispositif suivant la revendication 12, caractérisé en ce que plusieurs sources de lumière UV (22) sont disposées, les unes à côté des autres, en une ou plusieurs rangées perpendiculaires à la direction d'écoulement (B), les sources de lumière UV étant regroupées, comme sous-ensemble, dans un module radiateur (44) .

**14.** Dispositif suivant revendication 13, caractérisé en ce que des modules radiateurs (44) sont montés à distance le long du canal 40.

FIG.1

FIG.2

FIG. 3

FIG. 4

FIG. 6

FIG. 5